# EUROPEAN PATENT APPLICATION

(11) **EP 4 317 977 A1**
(43) Date of publication of application: **07.02.2024**
(21) Application number: 22779623.2
(22) Date of filing: 17.02.2022
(51) Int. Cl.: G01N 33/543, G01N 21/78

(54) **TEST DEVICE AND CARTRIDGE**

(30) Priority: 30.03.2021 JP 2021057591
(71) Applicant: FUJIFILM Corporation, Tokyo 106-8620 (JP)
(72) Inventor: IKAMI, Seishi, Ashigarakami-gun, Kanagawa 258-8538 (JP)
(74) Representative: Hughes, Andrea Michelle
(86) International application number: PCT/JP2022/006452
(87) International publication number: WO 2022/209396

(57) **Abstract**

An assay apparatus used for an immunochromatographic assay, the assay apparatus including a loading part in which a cartridge is loaded, where the cartridge includes a carrier having an assay region in which a color development state changes depending on whether a sample is positive or negative, and a case in which the carrier is accommodated in an inner space defined by an outer shell formed of a translucent material and in which an opening portion for observing an observation region including the assay region is formed in a part of the outer shell, an illumination unit that has an emission end portion for emitting light that illuminates the observation region, where the illumination unit makes it possible to dispose at least a part of the emission end portion, in a state of being brought into contact, in a position separated from the opening portion on an outer surface of the outer shell of the cartridge in a loaded state, the position being capable of guiding a light incident into the outer shell from the emission end portion to an inner edge of the opening portion by transmitting the incident light through the outer shell, and a detection unit that is disposed at a position facing the opening portion of the cartridge in a loaded state and detects a color development state of the assay region, where the detection unit optically detects the color development state by receiving a reflected light which is emitted from an inner edge of the opening portion and reflected by a surface of the observation region.

## Description

### BACKGROUND OF THE INVENTION

### 1. Field of the Invention

The technique of the present disclosure relates to an assay apparatus and a cartridge used for an immunochromatographic assay.

### 2. Description of the Related Art

JP2013-238543A describes an assay device (referred to as a test piece in JP2013-238543A) for performing an assay of whether a sample is positive or negative, that is, whether or not the sample contains a test substance, using an immunochromatographic method. Such an assay device is called an assay cartridge or the like. The assay cartridge includes a carrier having an assay region for assaying whether a sample is positive or negative, and a case for accommodating the carrier. An antibody that reacts with the test substance is immobilized in the assay region, and the color development state of the assay region changes in a case where the antibody reacts with the test substance. In the case, an opening portion for observing the color development state of the assay region from the outside is formed.

On the other hand, JP2002-267664A describes a measuring device for an immunochromatographic test piece. In the measuring device, an immunochromatographic test tool, that is, a case, which accommodates an immunochromatographic test piece as a carrier is loaded. The light of the light emitting element is incident into the case loaded in the measuring device from the back surface side of the case via the light guide portion. In addition, a linear image sensor as a detection unit for measuring a change in a sample on the surface of the carrier is provided on the front surface side of the case. The light of the light emitting element is incident on the carrier from the back surface side of the carrier, and the light incident on the carrier is transmitted through the assay region. The detection unit receives the transmitted light transmitted through the carrier including the assay region.

### SUMMARY OF THE INVENTION

In the assay cartridge described in JP2013-238543A, the side wall of the opening portion is an inclined surface formed in a tapered shape. In a state where the assay cartridge is loaded in the assay apparatus, a detection unit that detects the reflected light reflected in the assay region is disposed at a directly-facing position directly facing the opening portion. In addition, a light source is disposed on the side of the detection unit, and the light source is located obliquely upward with respect to the opening portion. The light from the light source is emitted from obliquely upward with respect to the opening.

In a case where the detection unit is disposed at a directly-facing position directly facing the opening portion as described in JP2013-238543A, the opening portion must be irradiated with light from an oblique direction. In the case of being irradiated with light from an oblique direction in this way, there has been a problem that the light quantity distribution in the observation region tends to be uneven, caused by that a difference in distance from the light source occurs in the observation region including the assay region and the peripheral region thereof, that the light is vignetted in the periphery of the opening portion, or the like. The configuration of the assay apparatus described in JP2013-238543A is not sufficient to solve such a problem.

On the other hand, as described in JP2002-267664A, in a case where light is emitted toward the assay region of the carrier from the back surface side opposite to the front surface where the opening is disposed, the light emitting element can be disposed at a directly-facing position of the assay region. Therefore, the problem that the assay apparatus described in JP2013-238543A has is unlikely to occur.

However, there is a problem that since the detection unit receives the transmitted light transmitted through the carrier in the irradiation from the back surface side of the carrier, the detection unit is susceptible to an adverse effect due to an individual difference of the carrier. For example, in a case where, in a carrier, in a case where the stain adheres and the impurities are mixed inside the carrier on the deeper side than the assay region formed on the front surface, the light received by the detection unit includes unnecessary information other than the assay region, such as stain and impurities in a case where light is transmitted from the back surface side of the carrier.

In consideration of the above-described facts, the technique according to the present disclosure provides an assay apparatus and a cartridge that are less susceptible to adverse effects due to individual differences of the carrier and can suppress unevenness of the light quantity distribution in the observation region.

The assay apparatus of the present disclosure is an assay region used for an immunochromatographic assay includes a loading part in which a cartridge is loaded, where the cartridge includes a carrier having an assay region in which a color development state changes depending on whether a sample is positive or negative, and a case in which the carrier is accommodated in an inner space defined by an outer shell formed of a translucent material and in which an opening portion for observing an observation region including the assay region is formed in a part of the outer shell, an illumination unit that has an emission end portion for emitting light that illuminates the observation region, where the illumination unit makes it possible to dispose at least a part of the emission end portion in a state of being brought into contact, in a position separated from the opening portion on an outer surface of the outer shell of the cartridge in a loaded state, the position being capable of guiding a light incident into the outer shell from the emission end portion to an inner edge of the opening portion by transmitting the incident light through the outer shell, and a detection unit that is disposed at a position facing the opening portion of the cartridge in a loaded state and detects a color development state of the assay region, where the detection unit optically detects the color development state by receiving a reflected light which is emitted from an inner edge of the opening portion and reflected by a surface of the observation region.

In the assay apparatus of the present disclosure, a suppression part for suppressing light leakage in which light emitted from the emission end portion is not incident on the outer shell and leaks to the periphery of the emission end portion, is preferably provided.

In the assay apparatus of the present disclosure, preferably, the emission end portion has an emission window that emits light and a window frame that surrounds the periphery of the emission window, and the suppression part is a light shielding elastic member that is provided on the window frame and elastically deforms in a case of being pressed against the outer surface of the outer shell.

In the assay apparatus of the present disclosure, a contact position of the emission end portion is preferably an outer surface facing the detection unit in the outer shell.

In the assay apparatus of the present disclosure, it is preferable that the assay apparatus has at least two illumination units and the two illumination units are disposed on both sides of the opening portion in the outer shell one by one.

In the assay apparatus of the present disclosure, a light source of the illumination unit is preferably a semiconductor light source.

In the cartridge of the present disclosure, a translucent material having a diffusibility to diffuse light is preferably used as the translucent material.

In the cartridge of the present disclosure, a diffused light ray transmittance of the translucent material is preferably 0.1% or more and 50% or less in light having at least a specific wavelength in a case where a transmission distance is 2 mm.

In the cartridge of the present disclosure, a diffused light ray transmittance of the translucent material is preferably 1% or more and 40% or less in light having a wavelength of 420 nm to 680 nm in a case where a transmission distance is 2 mm.

In the cartridge of the present disclosure, the case preferably includes a light guide member that is formed of a translucent material having a higher light transmittance than the translucent material forming the outer shell, where the light guide member guides light incident from the emission end portion to the inner edge of the opening portion.

In the cartridge of the present disclosure, it is preferable that the light guide member is disposed on the outer surface of the outer shell and is disposed in contact with at least a part of the emission end portion of the illumination unit, and in the light guide member, a region other than a contact portion to be brought in contact with the emission end portion is shielded from light.

The assay apparatus and the cartridge of the present disclosure are less susceptible to adverse effects due to individual differences of the carrier and can suppress unevenness of the light quantity distribution in the observation region.

### BRIEF DESCRIPTION OF THE DRAWINGS

Fig. 1 is a perspective view showing an appearance of an assay apparatus according to the present disclosure.
Fig. 2 is a perspective view of an assay cartridge loaded into the assay apparatus according to the present disclosure.
Fig. 3 is an exploded perspective view of the assay cartridge according to the present disclosure.
Fig. 4A is a cross-sectional view showing a state in which a first pressing operation part of the assay cartridge according to the present disclosure is operated, and Fig. 4B is a cross-sectional view showing a state in which the first pressing operation part and a second pressing operation part are operated.
Fig. 5 is a side view showing a positional relationship between an assay strip, a multifunctional member, a first reagent holding part, and a second reagent holding part, in the assay cartridge according to the present disclosure.
Fig. 6 is an explanatory diagram of an immunochromatographic method.
Fig. 7 is a partially broken side view of the assay apparatus in a state where the assay cartridge according to the present disclosure is loaded.
Fig. 8 is a cross-sectional view showing a positional relationship between the assay cartridge according to the present disclosure and an illumination unit in the assay apparatus.
Fig. 9 is a cross-sectional view showing the illumination unit in the assay apparatus according to the present disclosure.
Fig. 10 is a cross-sectional view showing a path of light in an outer shell of a case forming the assay cartridge according to the present disclosure.
Fig. 11 is a cross-sectional view showing a comparative example.
Fig. 12A is a graph showing an example of the diffused light ray transmittance of a translucent material forming a cover member forming the assay cartridge according to the present disclosure, and Fig. 12B is a graph showing a different example.
Fig. 13 is a cross-sectional view showing a modified example in which the illumination unit is disposed on the inclined part.
Fig. 14A is a cross-sectional view showing a modified example in which a light guide member is provided in the assay cartridge according to the present disclosure, and Fig. 14B is a cross-sectional view showing a variation in a position of the light guide member.
Fig. 15A is a cross-sectional view showing a modified example in which a suppression part for suppressing light leakage from an illumination unit is formed by a screen, Fig. 15B is a cross-sectional view showing a modified example in which a recess is formed in a case to be the suppression part, and Fig. 15C is a cross-sectional view showing a modified example in which the suppression part is not formed.

### DESCRIPTION OF THE PREFERRED EMBODIMENTS

Hereinafter, an assay cartridge according to an embodiment of the present invention will be described with reference to the drawings. The constituent elements indicated by the same reference numerals in the drawings mean the same constituent elements. However, unless otherwise specified in the specification, each component is not limited to one, and a plurality of each component may be present.

In addition, description of overlapping configurations and reference numerals in the respective drawings may be omitted. The present invention is not limited to the following embodiments, and can be implemented with appropriate modifications, such as omitting a configuration or replacing a configuration with a different configuration within the scope of the object of the present invention.

The directions indicated by the arrows X and Y, which are appropriately shown in the respective figures, are directions along the horizontal plane and are orthogonal to each other. In addition, the direction indicated by the arrow Z is a direction along the perpendicular direction (vertical direction). The directions indicated by the arrows X, Y, and Z in respective figures coincide with each other.

### <Overview of immunochromatographic assay apparatus and assay cartridge>

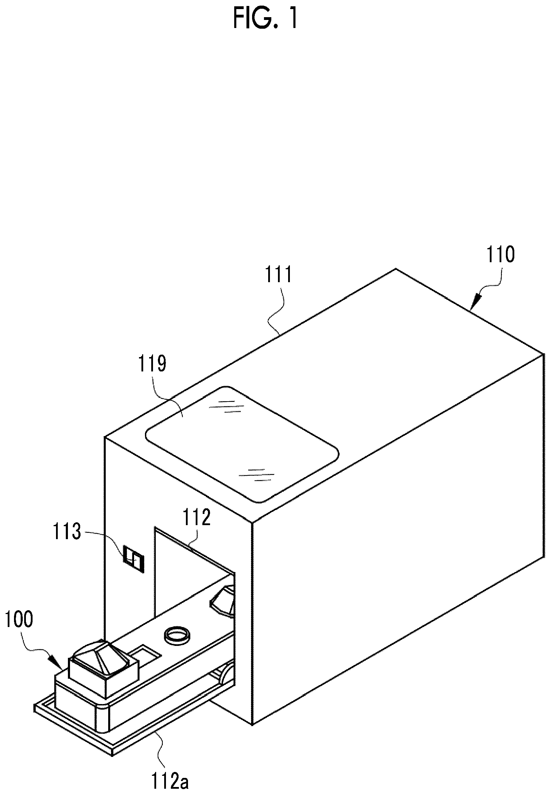

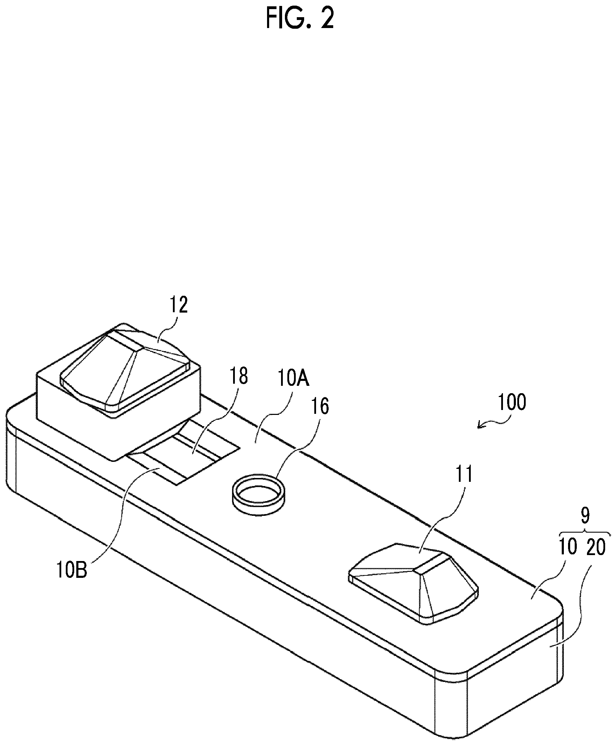

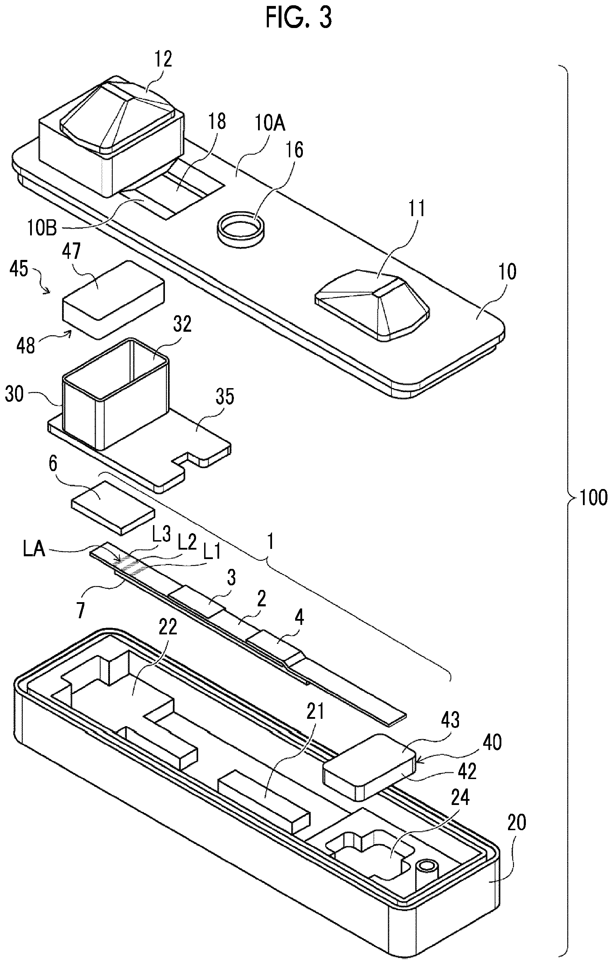

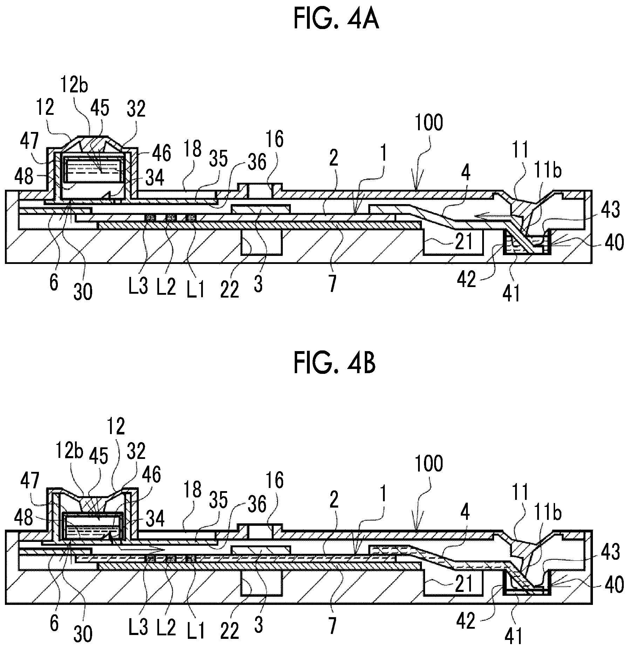

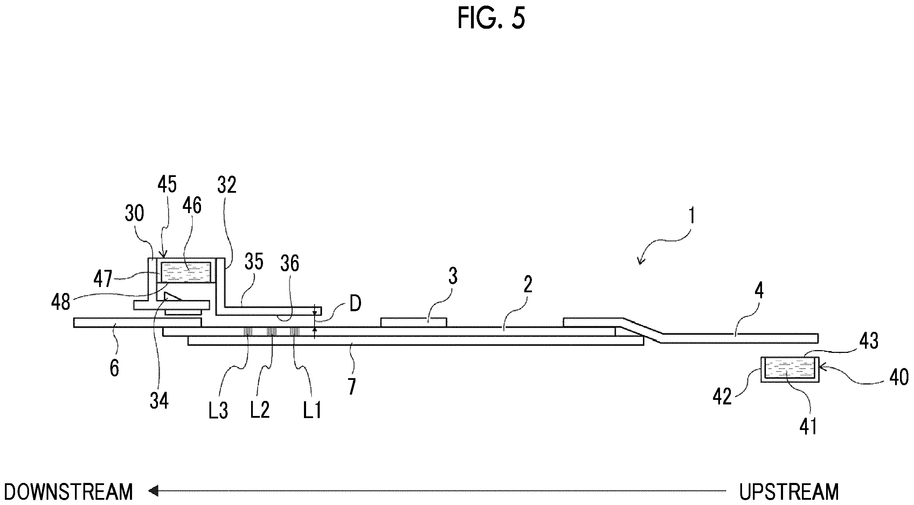

Embodiments of the immunochromatographic assay apparatus of the present disclosure will be described with reference to the drawings. Fig. 1 is a perspective view illustrating the appearance of the immunochromatographic assay apparatus 110 (hereinafter, simply referred to as an assay apparatus 110) according to one embodiment. Fig. 2 is an external view of a cartridge 100 mounted on the assay apparatus 110 and Fig. 3 is an exploded perspective view of the cartridge 100. Fig. 4 is a diagram showing a state in which the first pressing operation part 11 and the second pressing operation part 12 provided in the cartridge 100 are operated. Fig. 5 is a diagram showing the positional relationship of the main accommodated components in the cartridge 100.

The cartridge 100 is a single-use type that is used one by one for each sample of assay target. As shown in Fig. 3, an assay strip 1 including an immunochromatographic carrier 2 (hereinafter, referred to as a carrier 2) is provided in the cartridge 100. An assay region L1 is provided in the carrier 2, and the color development state changes depending on whether or not the sample contains a test substance, that is, whether the sample is positive or negative.

The "change in color development state" includes any of an aspect in which a first color different from the color of the carrier 2 changes to another second color (that is, a color change), an aspect in which the color of the carrier 2 changes to another color by developing a color different from that of the carrier 2 (that is, color development), and an aspect in which the density of the color changes (that is, a change in density).

The sample is simply required to be a specimen that may contain a test substance, and is not particularly limited. The sample is, for example, a biological specimen, particularly body fluid or excrement of an animal (particularly, a human) such as blood, serum, blood plasma, spinal fluid, tear fluid, sweat, urine, pus, nasal mucus, nasal swab, throat swab, nasal aspirate, or sputum, an organ, a tissue, a mucous membrane and skin, or swabs containing them, or a liquid specimen containing animals and plants themselves or a dried body thereof. Examples of the test substance include an antigen, an antibody, a protein, and a low-molecular-weight compound.

In the assay apparatus 110 of the present example, the cartridge 100 in a state in which the sample is spotted is loaded. Then, the assay apparatus 110 detects a color development state of the assay region L1 of the loaded cartridge 100, and presents the result of whether the sample is positive or negative. In a case of where a plurality of samples are assayed, the cartridge 100 for each sample is loaded one by one into the assay apparatus 110.

Hereinafter, the cartridge 100 will be described on the premise that the cartridge 100 is loaded into the assay apparatus 110. However, the cartridge 100 of the present example has a configuration that a user can confirm visually whether the sample is positive or negative without using the assay apparatus 110. Such a cartridge 100 is also referred to as an immunochromatographic assay tool, an immunochromatographic assay kit, or the like. Hereinafter, first, the configuration and the function of the cartridge 100 will be described, and then the assay apparatus 110 will be described.

### <Assay cartridge>

As shown in Fig. 2 and Fig. 3, as an example, the cartridge 100 includes a case 9 constituted of a case body 20 and a cover member 10. The case 9 has an elongated shape as a whole in accordance with the elongated shape of the assay strip 1.

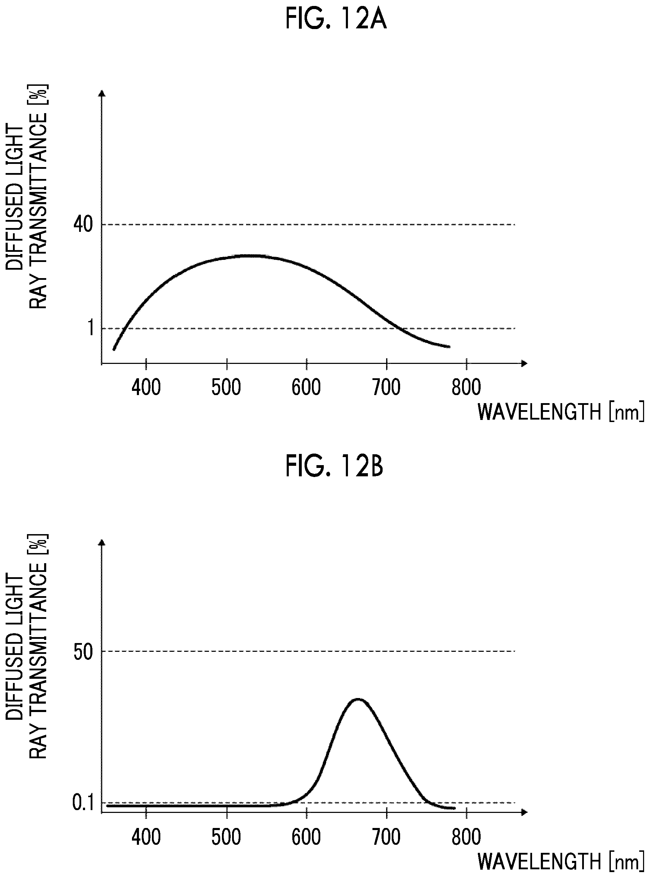

As will be described later, in a case of performing an assay in the assay apparatus 110, the cover member 10 is used as a light guide member that guides the illumination light that emitted to the cartridge 100. Therefore, the cover member 10 is formed of a translucent material having a diffusibility of diffusing light. As shown in Fig. 12A as an example, the diffused light ray transmittance of the translucent material used for the cover member 10 is 1% or more and 40% or less in visible light having a wavelength of 420 nm to 680 nm in a case where the transmission distance is 2 mm.

The "diffused light ray transmittance" of the translucent material is the proportion of a diffusion component that is diffused in the translucent material and is emitted from the translucent material in the incident light ray incident on the translucent material from the light source. A part of the incident light ray travels straight through the translucent material. The proportion at which this straight traveling component is emitted from the translucent material is called "parallel light ray transmittance". The parallel light ray transmittance of the translucent material forming the cover member 10 is, as a sample, 0.1% or less in visible light having a wavelength of 420 nm to 680 nm. In Fig. 12A, the diffused light ray transmittance in the wavelength range of 420 nm to 680 nm is at least 10% or more in the entire range. Therefore, the fact that the parallel light ray transmittance is 0.1% or less means that most of the light rays transmitted through the translucent material are diffusion components in the entire wavelength range of 420 nm to 680 nm.

Furthermore, the total value of the "diffused light ray transmittance" and the "parallel light ray transmittance" is referred to as a "total light ray transmittance". That is, the "total light ray transmittance" of the translucent material is the proportion of the total light ray emitted by being transmitted through the translucent material in the incident light rays incident on the translucent material from the light source.

The total light ray transmittance can be measured using, as an example, an integrating sphere. As is well known, the integrating sphere is a hollow sphere whose inner wall surface is composed of a reflecting surface, and is a measuring instrument for spatially integrating a straight traveling component and a diffusion component of light rays transmitted through a translucent material by reflection on the inner wall surface. By measuring the light quantity integrated in the integrating sphere, the light quantity of light rays transmitted through the translucent material can be measured.

The total light ray transmittance is measured while changing the wavelength of the light to be measured in a range of, for example, about 400 nm to 800 nm. On the other hand, in the parallel light ray transmittance, only the straight traveling component of the incident light ray that travels straight through the translucent material is measured without using an integrating sphere. For example, in a measuring device for measuring the light quantity, the straight traveling component can be measured by applying a mask to the light-receiving surface of the light-receiving part such that only the straight traveling component of the transmitted light ray is incident on the light-receiving part. As a matter of course, the parallel light ray transmittance is also measured while changing the wavelength of the light to be measured in a range of about 400 nm to 800 nm. The diffused light ray transmittance is a value obtained by subtracting the parallel light ray transmittance from the total light ray transmittance measured in this manner. The graph shown in Fig. 12A shows the diffused light ray transmittance calculated in this manner. The transmission distance is a distance in which the incident light rays transmit through the translucent material, and is, as an example, a thickness of the plate-shaped translucent material in a case where the translucent material has a plate shape. The transmission distance of 2 mm means that the thickness of the translucent material used for measuring the total light ray transmittance is 2 mm. The translucent material is, as an example, a resin material.

The light transmission characteristic as shown in Fig. 12A is a light transmission characteristic of a resin material, which is so-called milky white. As long as the resin material has the light transmission characteristics as shown in Fig. 12A, white light can be used as the illumination light, green light having a center wavelength of about 550 nm can be used as the illumination light, or red light having a center wavelength of about 650 nm can be used as the illumination light.

The case body 20 is formed of, for example, a resin material, an opening is formed in an upper part of the case body 20, and in addition to the assay strip 1, a first reagent holding part 40, a second reagent holding part 45, and the like are accommodated therein. The cover member 10 covers the opening of the case body 20 by being attached to the opening part of the case body 20. Accordingly, the assay strip 1 provided with the carrier 2 is accommodated in the internal space of the case 9.

The "outer shell" of the case 9 means the case body 20 and the cover member 10 themselves, which constitute the case 9, and is used to distinguish from the internal space of the case 9. That is, the case of the inside of the case 9 means an internal space, but the inside of the outer shell of the case 9 means the inside of each of the members constituting the case 9, that is, the cover member 10 and the case body 20. Similar to the case body 20, the cover member 10 is an outer shell of the case 9, and defines an internal space of the case 9 together with the case body 20. As described above, the cover member 10 is a translucent material. Therefore, the case 9 is a case in which the carrier 2 is accommodated in an internal space defined by a cover member 10 which is an outer shell formed of a translucent material.

In the present example, the cover member 10 that constitutes the upper part of the case 9 is provided with a dropping port 16, an observation window 18, a first pressing operation part 11, and a second pressing operation part 12. Each of these parts is integrally molded with the cover member 10 as an example. The dropping port 16 is an opening for adding dropwise a sample into the inside of the case 9. A boss is vertically provided on the edge of the dropping port 16 toward the upper part.

### (Observation window)

The observation window 18 is an opening portion for observing the assay region L1 from the outside. In the present example, the size of the observation window 18 is a size such that, in addition to the assay region L1, the control region L2 and the color development region L3, which will be described later, can also be observed. In the present specification, a region that includes the assay region L1, the control region L2, the color development region L3, and the peripheral region thereof and that can be observed from the observation window 18 is referred to as an observation region LA.

An inclined portion 10B forming a recess that falls from the plane portion 10A of the cover member 10, is provided around the observation window 18 of the cover member 10. The inclined portion 10B is a portion inclined obliquely downward with respect to the plane portion 10A which is a plane parallel to the surface of the observation region LA in the assay strip 1. The inclined portions 10B are formed on both sides of the observation window 18. The cover member 10 is an outer shell of the case 9, and the plane portion 10A and the inclined portion 10B are outer surfaces of the outer shell of the case 9.

### (First pressing operation part, second pressing operation part)

As shown in Fig. 2, Fig. 3, and Fig. 4, the first pressing operation part 11 is an operating part operated to supply the first reagent 41 in the first reagent holding part 40 to the carrier 2. The second pressing operation part 12 is an operating part operated to supply the second reagent 46 in the second reagent holding part 45 to the carrier 2. As will be described later, the first reagent 41 and the second reagent 46 are amplifying liquids for amplifying the color development in the assay region L1 in a case where the sample is positive.

As shown in Fig. 4A, in a case where a pressing force is applied from the outside as an external force to the first pressing operation part 11 by a pressing operation by a user or the like, the first pressing operation part 11 is deformed. As shown in Fig. 2, as an example, the first pressing operation part 11 has a quadrangular pyramid shape, and in a case where a pressing force is applied from above to a region including the apex of the quadrangular pyramid, as shown in Fig. 4A, the first pressed part 11 is deformed such that the apex of the quadrangular pyramid sinks into the inside of the case 9. In a case of where the first pressing operation part 11 is deformed in this manner, a pressing force is applied to the first reagent holding part 40 inside the case 9. In the first reagent holding part 40, deformation or the like due to a pressing force applied through the first pressing operation part 11 occurs. Due to this deformation or the like, the first reagent 41 held by the first reagent holding part 40 is supplied to the assay strip 1.

In addition, the first pressing operation part 11 is deformed by pressing and then the deformed state is maintained. Accordingly, after the first pressing operation part 11 is pressed, the supply of the first reagent 41 to the assay strip 1 is continued.

Similarly, as shown in Fig. 4B, in a case where a pressing force is applied from the outside as an external force to the second pressing operation part 12, the second pressing operation part 12 is deformed. Similarly to the first pressing operation part 11, the second pressing operation part 12 of the present example also has a quadrangular pyramid shape, and in a case where a pressing force is applied from above to a region including the apex of the quadrangular pyramid, the second pressing operation part 12 is deformed such that the apex of the quadrangular pyramid sinks into the inside of the case 9. In a case of where the second pressing operation part 12 is deformed in this manner, a pressing force is applied to the second reagent holding part 45 inside the case 9. In the second reagent holding part 45, deformation or the like due to a pressing force applied through the second pressing operation part 12 occurs. Due to this deformation or the like, the second reagent 46 held by the second reagent holding part 45 is supplied to the assay strip 1. In the second pressing operation part 12 of the present example, an abutting part 12b that abuts on the second reagent holding part 45 is provided.

### (First reagent holding part)

As shown in Fig. 3, the case body 20 accommodates the assay strip 1 including the carrier 2 along the longitudinal direction. As shown in Fig. 3 and Fig. 4, in the case body 20, the first reagent holding part 40 is disposed on one end part side (upstream side shown in Fig. 5) in the longitudinal direction. In the case body 20, in a portion where the first reagent holding part 40 is disposed, the first accommodating part 24 that is a recess-shaped in accordance with the shape of the first reagent holding part 40 is formed. One end part of the assay strip 1 is disposed above the first reagent holding part 40 in a state of being accommodated in the first accommodating part 24.

As shown in Fig. 4 and Fig. 5, the first reagent holding part 40 holds the first reagent 41. The first reagent holding part 40 is constituted of, for example, a container 42 formed of a resin material and having an opening on one surface, and a sheet member 43 that covers the opening of the container 42 and is breakable. The container 42 is filled with the first reagent 41, and the opening of the container 42 is sealed by the sheet member 43. The first reagent holding part 40 is disposed in the first accommodating part 24 in a posture in which the sheet member 43 faces upward.

The pressing force applied from the first pressing operation part 11 is transmitted to the sheet member 43 of the first reagent holding part 40 via the end part of the assay strip 1 to break the sheet member 43. The sheet member 43 is broken to immerse the end part of the assay strip 1 in the container 42, and thus the first reagent 41 is supplied to the assay strip 1. In the first pressing operation part 11 of the present example, a protruding part 11b that abuts on the sheet member 43. The protruding part 11b has, for example, an elongated shape extending in the longitudinal direction in the width direction of the assay strip 1 and a pointed shape toward the sheet member 43, such that the sheet member 43 is easily broken.

### (Multifunctional member)

In addition, the cartridge 100 includes a multifunctional member 30 having a function of accommodating the second reagent holding part 45. The multifunctional member 30 is disposed on the other end part side (downstream side shown in Fig. 5) of the case body 20 and above the assay strip 1. The multifunctional member 30 is a member in which the second accommodating part 32 and the flow channel forming part 35 are integrally formed. The second accommodating part 32 is a part accommodating the second reagent holding part 45. The second accommodating part 32 has a box shape having an opened upper surface. As shown in Fig. 5, on the bottom of the second accommodating part 32, a protrusion 34 for breaking a sheet member 48, which will be described later, of the second reagent holding part 45, and an opening that allows to flow the second reagent 46 flowed out from the second reagent holding part 45, toward the assay strip 1.

Furthermore, the flow channel forming part 35 is provided to be connected to the upstream side from the second accommodating part 32. The flow channel forming part 35 has a flat plate shape, is disposed at a position facing the assay region L1 or the like in the longitudinal direction of the assay strip 1, and is disposed with an interval from the assay strip 1. Then, between the flow channel forming part 35 and the assay strip 1, a flow channel for flowing the second reagent 46 flowed out from the second accommodating part 32 toward the assay region L1 or the like is formed. In addition, the flow channel forming part 35 is disposed between the observation window 18 and the assay region L1 or the like of the assay strip 1. Therefore, the flow channel forming part 35 is formed of a transparent member and thus the assay region L1 and the like can be observed through the observation window 18.

### (Second reagent holding part)

The second reagent holding part 45 holds the second reagent 46. The second reagent holding part 45 is constituted of, for example, a container 47 formed of a resin material and having an opening on one surface, and a sheet member 48 that covers the opening of the container 47 and is breakable. The container 47 is filled with the second reagent 46, and the opening of the container 47 is sealed by the sheet member 48. The second reagent holding part 45 is disposed in the second accommodating part 32 in a posture in which the sheet member 48 faces downward. Accordingly, the sheet member 48 faces the protrusion 34 in the second accommodating part 32.

The pressing force applied from the second pressing operation part 12 to the second reagent holding part 45 acts in a direction of pushing down the second reagent holding part 45 downwardly, whereby the sheet member 48 is pressed against the protrusion 34. The sheet member 48 is pressed against the protrusion 34 to break the sheet member 48. The sheet member 48 is broken, and thus the second reagent 46 is supplied to the assay strip 1 through the flow channel formed by the opening at the bottom of the second accommodating part 32 and the flow channel forming part 35.

As shown in Fig. 5, a gap (a clearance) D corresponding to the flow channel for the second reagent 46 is formed between a back surface 36 of the flow channel forming part 35 of the multifunctional member 30 and the carrier 2 of the assay strip 1. The gap D is, for example, in the range of 0.01 mm to 1 mm. The second reagent 46 flows out from the opening at the bottom of the second accommodating part 32 toward the carrier 2, and the second reagent 46 that has flowed out flows through the flow channel formed by the gap D and reaches at least above the assay region L1. The second reagent 46 that has reached on the assay region L1 infiltrates the assay region L1 from the flow channel.

An absorption pad 6 is disposed at an end part on the downstream side of the assay strip 1. As shown in Fig. 3, in the case body 20, a support part 22 that supports an end part of the assay strip 1 including the absorption pad 6 is formed at a position facing the absorption pad 6. A second accommodating part 32 of the multifunctional member 30 is disposed above the absorption pad 6. The support part 22 also supports the multifunctional member 30 via the absorption pad 6. In addition, in the case body 20, a support part 21 that supports a central part of the assay strip 1 is formed.

### <Assay strip>

The assay strip 1 includes a carrier 2, a liquid feeding pad 4, and an absorption pad 6. Then, the carrier 2 is fixedly supported on a back pressure-sensitive adhesive sheet 7.

### (Carrier)

The carrier 2 is a porous insoluble carrier for developing a sample, and includes an assay region L1, a control region L2, and a color development region L3. In addition, the carrier 2 includes a label holding pad 3. The label holding pad 3 constitutes a spotting region on which the sample is spotted from dropping port 16. The color development region L3 is disposed on the downstream side of the assay region L1 in a case where the direction toward the assay region L1 with respect to the spotting region is the downstream side of the carrier 2. In the present example, the assay region L1, the control region L2, and the color development region L3 are line-shaped regions extending in a direction perpendicular to the development direction of the sample in the carrier 2.

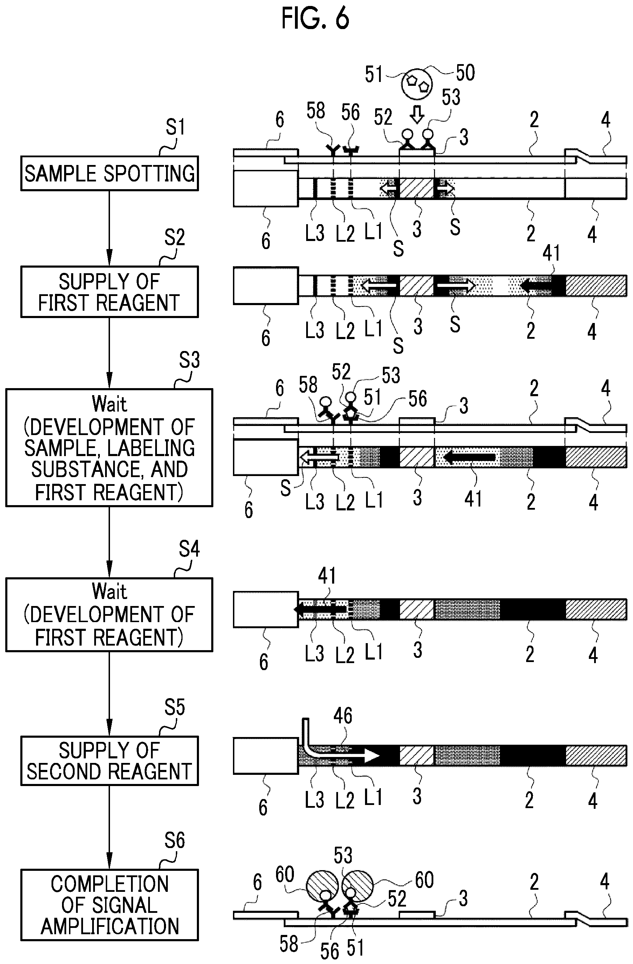

In Fig. 3 to Fig. 5, it shows a state in which the assay region L1, the control region L2, and the color development region L3 are expressed as lines, but these are not always expressed. Details will be described later, but before developing the sample 50 (see Fig. 6), the first reagent 41 (see Fig. 4 and Fig. 5), and the second reagent 46 (see Fig. 4 and Fig. 5), the colors of the assay region L1 and the control region L2 are substantially the same as the color of the carrier 2 (for example, white), and thus the assay region L1 and the control region L2 cannot be clearly visually recognized at this stage. The assay region L1 is expressed as a line by increasing the color optical density in a case where the sample 50 is developed and the developed sample 50 is positive. Since the color development of the assay region L1 is amplified by silver amplification, which will be described later, the assay region L1 develops a black color.

The control region L2 is also expressed as a line by increasing the color optical density in a case where the sample 50 is developed. Accordingly, the control region L2 becomes visible. Since the color development of the control region L2 is also subjected to silver amplification, the control region L2 also develops a black color.

On the other hand, only the color development region L3 is expressed and visible as a blackish dark green color (hereinafter, referred to as a dark green color) line even in a stage before the first reagent 41 is developed. However, the color development region L3 is expressed as an orange line by changing a dark green color to an orange color in a case where the first reagent 41 is developed.

As the carrier 2, for example, a porous material such as a nitrocellulose membrane can be used. In addition, the back pressure-sensitive adhesive sheet 7 on which the carrier 2 is fixed is a sheet-shaped substrate having a pressure-sensitive adhesive surface to which the carrier 2 is attached.

### (Carrier - label holding pad)

As shown in Fig. 6, a labeling substance 53 is fixed to the label holding pad 3. The labeling substance 53 is modified with the first binding substance 52 that specifically binds to the test substance 51 contained in the sample 50. The label holding pad 3 is fixed on the carrier 2 at a position facing the dropping port 16 (see Fig. 3) of the cover member 10. Therefore, the sample 50 is added dropwise onto the label holding pad 3 from the dropping port 16. Therefore, the label holding pad 3 corresponds to a spotting region on which the sample 50 is spotted.

The label holding pad 3 is fixed at a substantially center position in the longitudinal direction of the carrier 2. As the labeling substance 53, it is possible to use, for example, a gold colloidal particle having a diameter of 50 nm (EM. GC50, manufactured by BBI Solutions). The labeling substance 53 is not limited to the gold colloid, and a metal sulfide that can be used in a general chromatographic method, a coloring particle that is used in an immunoagglutination reaction, or the like can be used, where a metal colloid is particularly preferable. Examples of the metal colloid include a gold colloid, a silver colloid, a platinum colloid, an iron colloid, an aluminum hydroxide colloid, and a composite colloid thereof. In particular, at an appropriate particle diameter, a gold colloid is preferable since it exhibits a red color, a silver colloid is preferable since it exhibits a yellow color, the gold colloid is most preferable among them.

### (Carrier - assay region)

As shown in Fig. 6, the assay region L1 includes a second binding substance 56 that specifically binds to the test substance 51 and captures the test substance 51. In the assay region L1, in a case where the test substance 51 is captured by binding the second binding substance 56 to the test substance 51, the first binding substance 52 bonded to the test substance 51 and the labeling substance 53 are captured. In a case where the test substance 51 is included in the sample 50, the test substance 51 and the labeling substance 53 are captured in the assay region L1, and thus the color optical density in the assay region L1 is increased to be not less than a preset reference. The assay region L1 is a region for confirming the presence or absence of the test substance 51 by a labeling signal from the labeling substance 53 captured via the test substance 51.

### (Carrier - control region)

The control region L2 includes a third binding substance 58 that specifically binds to the first binding substance 52, and captures the labeling substance 53 via the first binding substance 52. In a case where the sample 50 is spotted on the label holding pad 3, the labeling substance 53 that is not bound to the test substance 51 among the labeling substances 53 modified with the first binding substance 52 is also developed in the carrier 2 toward the assay region L1 together with the sample 50. The labeling substance 53 that is not bound to the test substance 51 passes through the assay region L1 without being captured by the assay region L1. The labeling substance 53 that has passed through the assay region L1 is captured in the control region L2 via the first binding substance 52 by binding the first binding substance 52 to the third binding substance 58. The labeling substance 53 is captured in the control region L2, and thus the color optical density in the control region L2 is increased to be not less than a preset reference. The control region L2 is a region for confirming the completion of the development of the sample 50 by the labeling signal from the labeling substance 53 captured via the first binding substance 52. Therefore, the control region L2 may be referred to as a confirmation region.

### (Carrier - color development region)

The color development region L3 contains a substance whose color development state changes in response to the first reagent 41. The color development region L3 indicates that the first reagent 41 has been developed to that region by reacting with the first reagent 41 to develop a color or change a color. For example, in a case where a mixed aqueous solution of an iron nitrate aqueous solution and citric acid (manufactured by Fujifilm Wako Pure Chemical Corporation, product code 038-06925) is used as the first reagent 41, an aspect in which the color development region L3 is constituted of a color reagent immobilization line on which Bromocresol Green (manufactured by FUJIFILM Wako Pure Chemical Corporation) has been immobilized in a line shape is preferable. This aspect is the aspect of the color development region L3 of the present example. As described above, the color development region L3 of the present example is dark green color before reacting with the first reagent 41, and the dark green color is changed to an orange color in a case where the first reagent 41 reaches the color development region L3. The color development region L3 is sometimes referred to as an amplification index region because the timing of supplying the second reagent 46 after the first reagent 41 is developed is indicated by changing the color development state.

### (Binding substance)

The first binding substance 52 that modifies the labeling substance 53 and specifically binds to the test substance 51 is, for example, an antibody against the antigen in a case where the test substance is an antigen, or an antigen against the antibody in a case where the test substance is an antibody. In addition, in a case where the test substance is a protein, a low-molecular-weight compound, or the like, the first binding substance 52 is a substance that specifically binds to the test substance, such as an aptamer for the protein, the low-molecular-weight compound, or the like.

The second binding substance 56 that is fixed in the assay region L1 and specifically binds to the test substance 51 is, for example, an antibody against the antigen in a case where the test substance is an antigen, or an antigen against the antibody in a case where the test substance is an antibody. In addition, in a case where the test substance is a protein, a low-molecular-weight compound, or the like, the second binding substance 56 is a substance that specifically binds to the test substance, such as an aptamer for the protein, the low-molecular-weight compound, or the like. The first binding substance 52 and the second binding substance 56 may be the same as or different from each other.

The third binding substance 58 that specifically binds to the first binding substance 52 may be the test substance 51 itself or may be a compound having a site recognized by the first binding substance 52. Examples thereof include a compound obtained by binding a derivative of the test substance 51 to a protein, and the like.

For example, in a case where the test substance 51 is an influenza A virus or a biomarker thereof, anti-influenza A monoclonal antibody (product name: Anti-Influenza A SPTN-5 7307, Medix Biochemica) can be used as the first binding substance 52 and the second binding substance 56, and an anti-mouse IgG antibody (anti-mouse IgG (H+L), rabbit F(ab')2, product number 566-70621, manufactured by FUJIFILM Wako Pure Chemical Corporation) can be used as the third binding substance 58.

### (Liquid feeding pad)

The liquid feeding pad 4 is disposed in contact with one end of the carrier 2 and the first reagent 41 is fed to the carrier 2 from the upstream side of the spotting region constituted of the label holding pad 3. As shown in Fig. 4A, in a case where the first pressing operation part 11 is pressed, one end of the liquid feeding pad 4 is immersed in the first reagent holding part 40. The liquid feeding pad 4 is formed of a porous material and absorbs the first reagent 41, and the absorbed first reagent 41 is fed to the carrier 2 by a capillary action.

### (Absorption pad)

The absorption pad 6 is disposed in contact with the other end of the carrier 2 and absorbs the sample 50, the first reagent 41, and the second reagent 46, which are developed on the carrier 2. The absorption pad 6 is also formed of a porous material.

### <Amplifying liquid>

In the present embodiment, the first reagent 41 and the second reagent 46 are amplifying liquids that amplify the color development in the assay region L1 and the control region L2 by reacting with each other. In a case where a metal-based labeling substance such as a gold colloid is used as the labeling substance 53 as in the present example, for example, silver amplification is used as a method of amplifying the labeling signal of the labeling substance 53. The first reagent 41 and the second reagent 46 are, as an example, amplifying liquids used for silver amplification, and the reaction between the first reagent 41 and the second reagent 46 using the labeling substance 53 as a catalyst is an amplification reaction. By the amplification reaction, silver particles 60 (see Fig. 6) having a particle diameter relatively larger than that of the labeling substance 53 are generated.

More specifically, in the present example, the first reagent 41 is a reducing agent that reduces silver ions, and the second reagent 46 is a silver ion. In a case where the first reagent 41, which is a reducing agent, and the second reagent 46, which is a silver ion, are brought into contact with the labeling substance 53, silver particles 60 are generated, and the generated silver particles 60 deposits on the labeling substance 53 using the labeling substance 53 as a nucleus. By depositing the silver particles 60 on the labeling substance 53, silver particles 60 having a particle diameter larger than that of the labeling substance 53 (see Fig. 6) are generated. Accordingly, the labeling signal issued by the labeling substance 53 is amplified, and as a result, the color development of the labeling substance 53 is amplified in the assay region L1 and the control region L2.

### (First Reagent)

As the reducing agent as the first reagent 41, any inorganic or organic material or a mixture thereof can be used as long as the silver ion used as the second reagent 46 can be reduced to silver. Preferred examples of the inorganic reducing agent include a reducing metal salt and a reducing metal complex salt, of which the atomic valence is capable of being changed with a metal ion such as Fe²⁺, V²⁺, or Ti³⁺. In a case where an inorganic reducing agent is used, it is necessary to remove or detoxify oxidized ions by complexing or reducing the oxidized ions. For example, in a system in which Fe²⁺ is used as the reducing agent, a complex of Fe³⁺, which is an oxide, is formed using citric acid or ethylenediaminetetraacetic acid (EDTA), and therefore detoxification is possible. In the present system, such an inorganic reducing agent is preferably used, and it is more preferable that a metal salt of Fe²⁺ is preferably used.

In the reducing agent, a developing agent used in a light-sensitive silver halide photographic material of a wet-type (for example, methyl gallate, hydroquinone, substituted hydroquinone, 3-pyrazolidones, p-aminophenols, p-phenylenediamines, hindered phenols, amidoximes, azines, catechols, pyrogallols, ascorbic acid or a derivative thereof, and leuco dyes), and other materials obvious to those who are skilled in the related art in the present field, for example, a material described in US6020117A can be used.

As the reducing agent, an ascorbic acid reducing agent is also preferable. The useful ascorbic acid reducing agent includes ascorbic acid, an analogue thereof, an isomer thereof, and a derivative thereof. Preferred examples thereof include D- or L-ascorbic acid and a sugar derivative thereof (for example, γ-lactoascorbic acid, glucoascorbic acid, fucoascorbic acid, glucoheptoascorbic acid, or maltoascorbic acid), a sodium salt of ascorbic acid, a potassium salt of ascorbic acid, isoascorbic acid (or L-erythroascorbic acid), a salt thereof (for example, an alkali metal salt, an ammonium salt, or a salt known in the related technical field), ascorbic acid of the enediol type, ascorbic acid of the enaminol type, and ascorbic acid of the thioenol type. Particularly, D-ascorbic acid, L-ascorbic acid, or D,L-ascorbic acid (and an alkali metal salt thereof) or isoascorbic acid (or an alkali metal salt thereof) is preferable, and a sodium salt is a preferred salt. A mixture of these reducing agents can be used as necessary.

### (Second reagent)

The solution containing silver ions, which is used as the second reagent 46, is preferably a solution obtained by dissolving a silver ion-containing compound in a solvent. As the silver ion-containing compound, an organic silver salt, an inorganic silver salt, or a silver complex can be used. An inorganic silver salt or a silver complex is preferable. As the inorganic silver salt, it is possible to use a silver ion-containing compound having a high solubility in solvents such as water, and examples thereof include silver nitrate, silver acetate, silver lactate, silver butyrate, and silver thiosulfate. Silver nitrate is particularly preferable. The silver complex is preferably a silver complex in which silver is coordinated with a ligand having a water-soluble group such as a hydroxyl group or a sulfone group, and examples thereof include silver hydroxythioether.

### <Immunochromatographic method>

An immunochromatographic method will be described with reference to Fig. 6. Here, a case where the sample 50 includes the test substance 51, that is, on the premise that the sample 50 is positive will be described.

First, the sample 50 is spotted on the label holding pad 3 which is the spotting region (Step S1). The test substance 51 in the sample 50, which is spotted on the label holding pad 3, specifically binds to the first binding substance 52 that modifies the labeling substance 53 contained in the label holding pad 3. In the carrier 2, the sample 50 is developed on the downstream side from the label holding pad 3 in the carrier 2 by the capillary action. A part of the sample 50 is also developed on the upstream side. The arrow S indicates a state in which the sample 50 is developed.

Next, the first reagent 41 is supplied (Step S2). The first reagent 41 is supplied from the liquid feeding pad 4. The first reagent 41 is supplied to the carrier 2 via the liquid feeding pad 4 and is developed on the downstream side.

After that, the process waits until the first reagent 41 is developed on the downstream side (Step S3 and Step S4). "Wait" shown in Fig. 6 means an action of waiting. The first reagent 41 is gradually developed to the downstream side, and the sample 50 to be developed from the label holding pad 3 and the labeling substance 53 modified with the first binding substance 52 are developed to the downstream side to be pushed by the first reagent 41 (Step S3).

The test substance 51 in the sample 50 that has been developed to the downstream side and has reached the assay region L1 is captured by the second binding substance 56 of the assay region L1. That is, the labeling substance 53 bonded via the test substance 51 and the first binding substance 52 is captured in the assay region L1. On the other hand, the labeling substance 53 that is not bound to the test substance 51 passes through the assay region L1 without being captured and is captured by the third binding substance 58 of the control region L2.

In a case where the development of the first reagent 41 proceeds and the first reagent 41 reaches the color development region L3 (Step S4), the color development region L3 reacts with the first reagent 41 to change the color development state. In the present example, the color development region L3 is dark green color before reacting with the first reagent 41, and the dark green color is changed to an orange color by reacting with the first reagent 41.

After the first reagent 41 is sufficiently developed, the second reagent 46 is supplied to the carrier 2 (Step S5). The second reagent 46 is supplied to the carrier 2 from the downstream side of the color development region L3 and is developed on the upstream side. Here, the first reagent 41 is a first amplifying liquid containing a reducing agent that reduces silver ions, and the second reagent 46 is a second amplifying liquid containing silver ions. By reacting the first amplifying liquid with the second amplifying liquid, the silver particles 60 are generated using the gold colloidal particles that are the labeling substance 53 as a catalyst. Accordingly, the labeling signal is amplified (Step S6).

The configuration and functions of the cartridge 100 have been described so far, and a schematic configuration of the assay apparatus 110, a procedure for assay, and the like will be described below, and then a characteristic configuration of the assay apparatus 110 will be described.

### <Immunochromatographic assay apparatus>

Back to Fig. 1, the assay apparatus 110 includes a case body 111, and the case body 111 includes a cartridge loading part 112 in which the cartridge 100 is attachably and detachably loaded. As an example, an opening for inserting the cartridge 100 into the case body 111 and an opening and closing lid 112a for opening and closing the opening are provided on the front surface of the case body 111. The opening and closing lid 112a is opened when the cartridge 100 is loaded, and the cartridge 100 is inserted into the case body 111. In a case where the cartridge 100 is loaded into the cartridge loading part 112, the opening and closing lid 112a is closed. The assay is performed in a state where the opening and closing lid 112a is closed.

In addition, a power switch 113 is provided on the front surface of the case body 111, and a monitor 119 is provided on the upper surface of the case body 111. A determination result, an error message, and the like are displayed on the monitor 119. As an example, the monitor 119 is a touch panel monitor, and various operation screens are displayed. Through the operation screen, the user can input a start instruction of processing and an operation instruction such as selection of an assay procedure.

The assay apparatus 110, as an example, a cartridge 100 is loaded in a state in which the spotting of the sample on the carrier 2 and the supply of the first reagent 41 and the second reagent 46 to the carrier 2 is started. That is, in the present example, the pressing operation of the first pressing operation part 11 and the second pressing operation part 12 is performed by the user before the cartridge 100 is loaded into the assay apparatus 110. Accordingly, the cartridge 100 is in the state shown in Fig. 4B, and the cartridge 100 is loaded into the assay apparatus 110 in this state. The assay apparatus 110 detects a color development state of the assay region L1 of the loaded cartridge 100, and presents the result of whether the sample is positive or negative. In a case of where a plurality of samples are assayed, the cartridge 100 for each sample is loaded one by one into the assay apparatus 110.

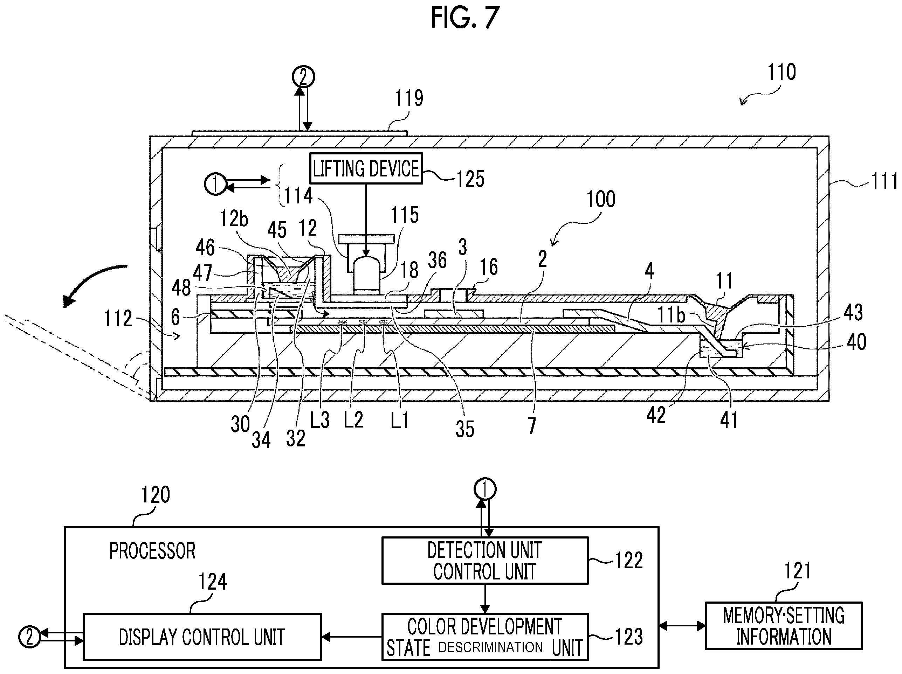

As shown in Fig. 7, in addition to the cartridge loading part 112, the assay apparatus 110 further includes a detection unit 114, an illumination unit 115, a lifting device 125, a processor 120, and a memory 121 in the case body 111. In Fig. 7, the processor 120 and the memory 121 are illustrated outside the case body 111 of the assay apparatus 110, but this is a schematic diagram, and the processor 120 and the memory 121 are actually disposed inside the case body 111.

The detection unit 114 is disposed at a position facing the observation window 18 of the cartridge 100 in a state of being loaded in the assay apparatus 110, and detects a color development state of the assay region L1 included in the observation region LA. This detection unit 114 optically detects the color development state of the assay region L1 by receiving the reflected light that is emitted from the inner edge 18A of the observation window 18 as the opening portion and that is reflected on the surface of the observation region LA.

The detection unit 114 is an image sensor such as a complementary metal oxide semiconductor (CMOS) image sensor and a charge coupled device (CCD) image sensor and images an observation region LA including an assay region L1, a control region L2, and a color development region L3. Then, the imaged image is output from the detection unit 114 to the processor 120.

The illumination unit 115 illuminates the observation region LA including the assay region L1, the control region L2, and the color development region L3 during imaging by the detection unit 114.

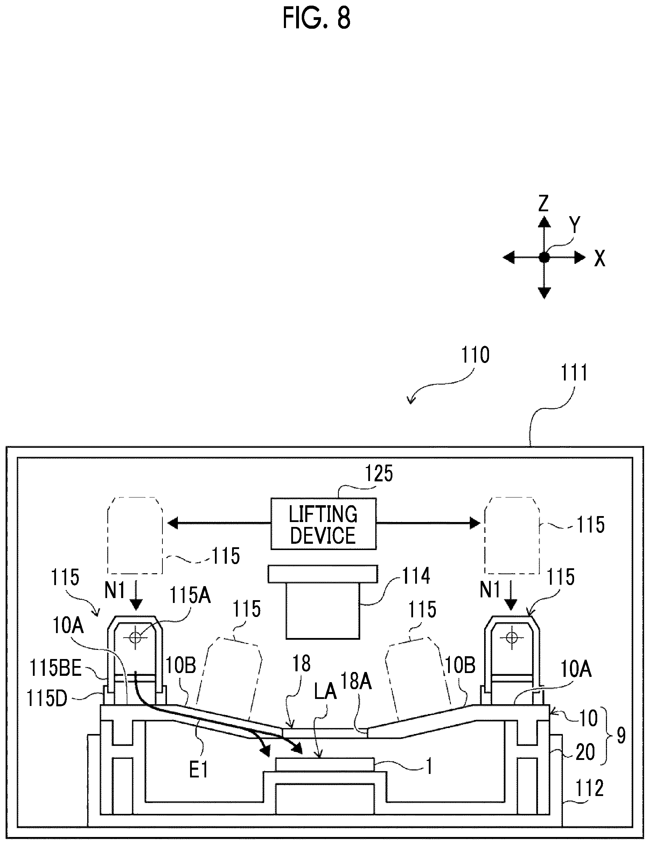

The lifting device 125 is an example of a moving mechanism that moves the illumination unit 115. In the illumination unit 115, the cover member 10 of the cartridge 100 is used as a light guide member for illumination light. The lifting device 125 moves the illumination unit 115 between a contact position where the illumination unit 115 is brought into contact with the outer surface of the cover member 10 which is a light guide member (see a position shown by a solid line in Fig. 8) and a retreat position where the illumination unit 115 is retracted from the contact position (see a position shown by a two-dot chain line in Fig. 8). The lifting device 125 is constituted of a motor, an actuator, and the like. At the time of loading and unloading the cartridge 100, the illumination unit 115 is moved to the retreat position by the lifting device 125.

The processor 120 integrally controls each part of the assay apparatus 110. An example of the processor 120 is a Central Processing Unit (CPU) that performs various types of control by executing a program. The CPU functions as a control unit having a detection unit control unit 122, a color development state discrimination unit 123, and a display control unit 124 by executing a program. The memory 121 is an example of a memory connected to or built in the CPU as the processor 120. For example, a control program is stored in the memory 121. The processor 120 is realized by the CPU executing a control program.

In addition to the control program, the memory 121 stores setting information that is preset in order for the processor 120 to perform various types of control. As the setting information, information necessary for the color development state discrimination unit 123 to discriminate a change in the color development state is recorded.

The detection unit control unit 122 controls the imaging timing of the observation region LA by the detection unit 114. In addition, the detection unit control unit 122 controls an operation of the lifting device 125.

The color development state discrimination unit 123 executes the assay region discrimination processing based on the detection signal output by the detection unit 114.

The assay region discrimination processing is a processing of discriminating presence or absence of a change in the color development state of the assay region L1 based on the detection signal output by the detection unit 114. In the present example, since a line is expressed in the assay region L1 by the labeling substance 53 being captured in the assay region L1, or the silver amplification after capturing the labeling substance 53, in the assay region discrimination processing, presence or absence of the expression of the line in the assay region L1 is discriminated.

In a case where the color development state discrimination unit 123 discriminates that the change in the color development state in the assay region L1 is present, the processor 120 determines that the sample 50 is positive. In this case, an indication that the assay result is "positive" is displayed on the monitor 119 via the display control unit 124. In addition, in a case where the color development state discrimination unit 123 discriminates that the change in the color development state in the assay region L1 is absent, the processor 120 determines that the sample 50 is negative. In this case, an indication that the assay result is "negative" is displayed on the monitor 119 via the display control unit 124.

### <Immunochromatographic assay>

The procedure of the immunochromatographic assay using the assay apparatus 110 of the present embodiment will be described simply. As described above, before loading the cartridge 100 into the assay apparatus 110, preprocessing such as spotting of the sample 50, supply of the first reagent 41, and supply of the second reagent 46 is performed by a user. The spotting of the sample 50 is performed by adding dropwise the sample 50 from the dropping port 16 of the cartridge 100 onto the spotting region of the carrier 2. The supply of the first reagent 41 is performed by the user pressing the first pressing operation part 11 of the cartridge 100. The supply of the second reagent 46 is performed by the user pressing the second pressing operation part 12 of the cartridge 100 after confirming that the color development region L3 has changed color from dark green to orange.

After such preprocessing is performed, the cartridge 100 is loaded into the assay apparatus 110. In a case where the cartridge 100 is loaded in the assay apparatus 110, the processor 120 moves the illumination unit 115 to the contact position by the lifting device 125 to light the illumination unit 115. Accordingly, the observation region LA of the cartridge 100 is illuminated. In this state, the processor 120 causes the detection unit 114 to perform imaging of the observation region LA. Then, the processor 120 determines the color development state of the assay region L1 based on the captured image of the observation region LA.

Then, in a case where it is discriminated that a change in the color development state of the assay region L1 is present, the processor 120 displays an indication that the assay result is "positive" on the monitor 119. In addition, in a case where it is discriminated that a change in the color development state of the assay region L1 is absent, the processor 120 displays an indication that the assay result is "negative" on the monitor 119.

### <Irradiation method of illumination light>

Hereinafter, an irradiation method of the illumination light, which is a characteristic of the assay apparatus 110, will be described with reference to Fig. 8 to Fig. 10 as appropriate. As described above, the detection unit 114 is disposed at a position facing the observation window 18 of the cartridge 100 in a state of being loaded in the assay apparatus 110. As shown in Fig. 8, more accurately, the detection unit 114 is disposed at a directly-facing position facing in front of the observation region LA. In addition, two illumination units 115 are provided, and disposed on both sides of the detection unit 114 in the width direction of the cartridge 100 one by one. The two illumination units 115 each move up and down at positions on both sides of the detection unit 114. The illumination unit 115 goes down from the retreat position indicated by the two-dot chain line toward the outer surface of the cover member 10 to move to a contact position in contact with the outer surface of the cover member 10.

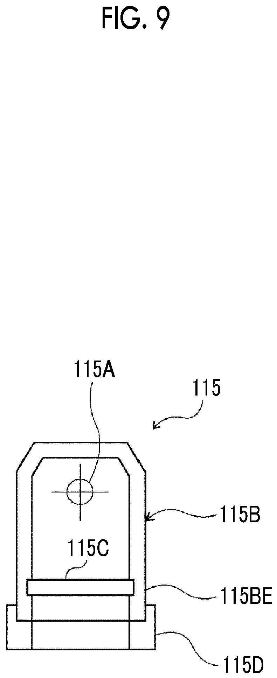

As shown in Fig. 9, the illumination unit 115 includes a light source 115A and a case 115B. The light source 115A is a semiconductor light source such as a light emitting diode. The case 115B is a cylindrical-shaped container that holds the light source 115A inside, and an opening for emitting light is formed at one end in the cylinder axis direction. The light emitted by the light source 115A is emitted from this opening. In the illumination unit 115, an end portion on a side where an opening of the case 115B is formed is an emission end portion 115BE that emits light that illuminates the observation region LA.

The emission end portion 115BE has an emission window 115C that emits light and a window frame that surrounds the periphery of the emission window 115C. The emission window 115C is, for example, a transparent cover that protects the light source 115A while transmitting the light of the light source 115A. The emission window 115C is formed of, for example, an acrylic resin or glass. The emission window 115C is fitted to an opening edge in which the opening is formed in the case 115B. The opening edge is formed in a circular shape to be capable of surrounding the periphery of, for example, a circular emission window 115C, and the opening edge functions as a window frame.

The light emitted from the light source 115A is emitted to the outside of the case 115B through the emission window 115C. The emission end portion 115BE of the present example is described in an example in which the emission window 115C is composed of a transparent cover, and a window frame are provided, but the present invention is not limited thereto. The emission window 115C composed of a transparent cover may not be provided, and in the emission end portion 115BE, an emission opening capable of emitting illumination light may be simply formed.

A light shielding member 115D is attached to the emission end portion 115BE. The light shielding member 115D is formed in an annular shape by an elastic member having a light shielding property such as black rubber, and is provided on the entire circumference of the window frame of the emission end portion 115BE. The light shielding member 115D functions as a suppression part for suppressing light leakage, as will be described later.

As shown in Fig. 8, the illumination unit 115 is disposed in a state where the emission end portion 115BE is contact with the outer surface of the outer shell of the case 9 of the cartridge 100 in a loaded state at a contact position (indicated by a solid line) with the cartridge 100. The contact position is a position separated from the observation window 18 on the outer surface of the cover member 10 which is the outer shell of the case 9, and a position where light incident into the inside of the cover member 10 from the emission end portion 115BE can be guided to the inner edge 18A of the observation window 18 by allowing the light to be transmitted inside of the cover member 10.

In the present example, the contact position of the emission end portion 115BE is set to the plane portion 10A of the cover member 10. The plane portion 10A constitutes an upper surface of the case 9 together with the inclined portion 10B, and both face the detection unit 114. That is, the contact position of the emission end portion 115BE is the outer surface facing the detection unit 114 in the outer shell of the case 9. "A position separated from the observation window 18", which is one of the requirements for the contact position, is a position distant from the observation window 18 in the plane portion 10A. Since the detection unit 114 is disposed in front of the observation window 18, the illumination unit 115 needs to be disposed at a position outside the field of view of the detection unit 114. Therefore, the contact position needs to be separated from the observation window 18.

In a case where the illumination unit 115 is in the contact position, the light emitted from the emission end portion 115BE is incident into the inside of the cover member 10. As indicated by the arrow E1, as an example, this light transmits through the inside of the cover member 10 which is an outer shell and is guided to the inner edge 18A of the observation window 18. As described above, since the cover member 10 is a translucent material having diffusibility, the light incident into the inside of the cover member 10 is guided while being diffused inside. Furthermore, the guided light is emitted from the inner edge 18A of the observation window 18 which is an opening portion to illuminate the observation region LA.

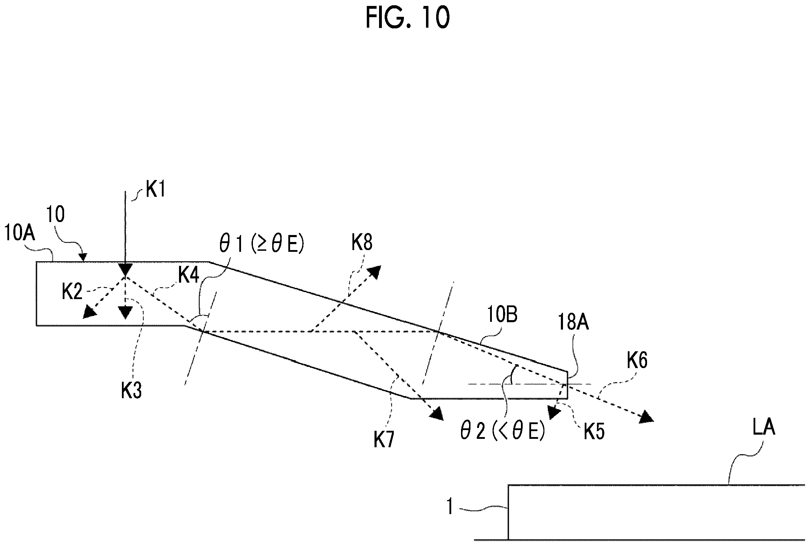

Here, Fig. 10 shows an example of a path of light incident into the inside of the cover member 10. As indicated by the arrow K1, the light incident into the inside of the cover member 10 is diffused in various directions inside the cover member 10 as indicated by the arrows K2, K3, and the straight line K4.

The diffused light shown by the straight line K4 is reflected on the inner surface of the cover member 10 and transmits through the inside of the cover member 10. Here, in a case where the incidence angle of the diffused light incident on the inner surface of the cover member 10 is equal to or greater than the critical angle θE as indicated by the angle θ1, the diffused light is totally reflected on the inner surface of the cover member 10. On the other hand, in a case where the incidence angle is smaller than the critical angle θE as indicated by the angle θ2, a part of the diffused light is reflected on the inner surface of the cover member 10 as indicated by the arrow K5, and the rest is emitted toward the outside of the cover member 10 as indicated by the arrow K6. The critical angle θE is determined according to the refractive index of the translucent material constituting the cover member 10. The shape and the translucent material of the cover member 10 are selected such that the emitted light quantity emitted from the inner edge 18A of the observation window 18 is large.

Since the light transmitted through the inside of the cover member 10 is diffused inside, the diffused light may be emitted from a portion other than the inner edge 18A of the observation window 18 as indicated by the arrows K7 and K8. Even in this case, as indicated by the arrow K7, a part of the light emitted from the peripheral portion of the observation window 18 can be used as the illumination light for illuminating the observation region LA.

On the other hand, as indicated by the arrow K8, the light emitted to the outside of the cover member 10 at a position relatively distant from the observation window 18 cannot be used as the illumination light of the observation region LA. In addition, it is also conceivable that such light is directly incident on the detection unit 114. Such light directly directed to the detection unit 114 is unnecessary light that causes noise for the detection unit 114. In order to block such unnecessary light, a light shielding material such as a light shielding coating material is preferably disposed on the outer surface of the cover member 10 except for a peripheral portion of the observation window 18 including the inner edge 18A.

In addition, in the emission end portion 115BE, the emission window 115C is disposed on the deeper side by one state than the light shielding member 115D toward the light source 115A. Therefore, in a case where the emission end portion 115BE is in the contact position, the light shielding member 115D, which is a part of the emission end portion 115BE, comes into contact with the plane portion 10A of the cover member 10. In a case where the light shielding member 115D is pressed against the plane portion 10A of the cover member 10, the light shielding member 115D is elastically deformed to be closely attached to the plane portion 10A. Accordingly, the light shielding member 115D functions as a suppression part for suppressing light leakage emitted from the emission end portion 115BE that does not enter the outer shell of the case 9 and leaks to the periphery of the emission end portion 115BE.

### <Action and effect>

In the assay apparatus 110 of the present disclosure, a cartridge 100 that accommodates the carrier 2 having the assay region L1 is loaded. The color development state of the assay region L1 of the carrier 2 changes depending on whether the sample is positive or negative, and the detection unit 114 detects this color development state.

In order to accurately detect the color development state of the assay region L1, the observation region LA including the assay region L1 is illuminated by the illumination unit 115, and the color development state, for example, the optical density, the color, and the like of the assay region L1 in the observation region LA need to be accurately grasped. Therefore, it is not preferable that the light quantity distribution in the observation region LA is uneven due to the partial occurrence of shadows, high-brightness portions, and the like in the observation region LA in a state of being illuminated by the illumination light. Therefore, it is preferable that the light quantity distribution in the observation region LA is as even as possible.

In the assay apparatus 110 of the present example, as shown in Fig. 8, the illumination unit 115 is disposed in a state where at least a part of the emission end portion 115BE from which light is emitted is in contact with the plane portion 10A that is an outer surface of the cover member 10, which is an outer shell of the cartridge 100 in a state of being loaded, at a position separated from the observation window 18. The contact position of the illumination unit 115 is a position capable of being guided to the inner edge 18A of the observation window 18 as an opening portion, as indicated by an arrow E1, by allowing the light incident into the inside of the cover member 10 from the emission end portion 115BE to be transmitted through the inside of the cover member 10. Therefore, in the assay apparatus 110, the light of the illumination unit 115 is guided to the inner edge 18A by being transmitted through the inside of the cover member 10, and the observation region LA is irradiated with the light emitted from the inner edge 18A as the illumination light.

Accordingly, the shadow of the cover member 10 is unlikely to be generated in the observation region LA. Therefore, it is possible to improve the evenness of the light quantity distribution in the observation region LA.

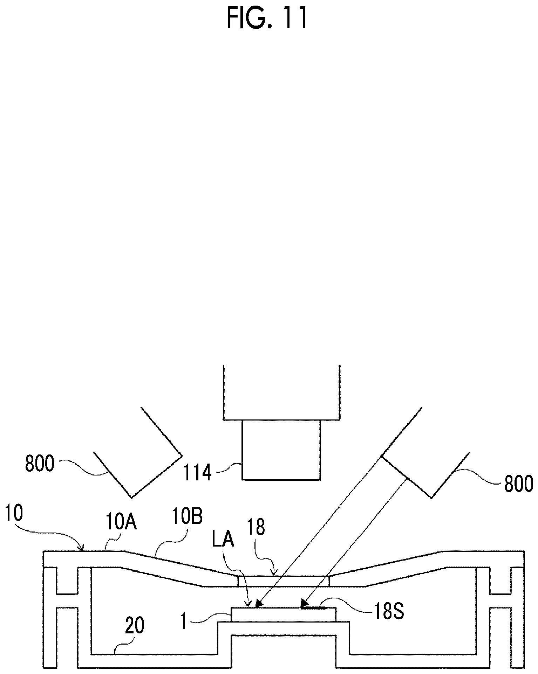

The reason is as follows. For example, consider the prior art of Fig. 11 as a comparative example. In the comparative example, similarly to the detection unit 114, an illumination unit 800 is disposed without coming into contact with the cover member 10 of the cartridge 100 and with an interval from the cover member 10. In a case where the observation region LA is illuminated from the illumination unit 800 in such an aspect, a shadow of the observation window 18 may be generated in the observation region LA. This is because the detection unit 114 is often disposed to directly face the observation region LA to accurately detect the color development state of the assay region L1 in the observation region LA. Therefore, the illumination unit 800 has no choice but to be disposed to avoid the detection unit 114, and for example, as shown in Fig. 11, the illumination unit 800 is disposed obliquely above the observation window 18. In a case where the observation region LA is illuminated from such a position, the observation region LA is illuminated from obliquely above the observation window 18, and thus the illumination light is eclipsed by the inner edge 18A of the observation window 18. Therefore, a shadow of the observation window 18 is generated in the observation region LA, and unevenness in the light quantity distribution is likely to occur in the observation region LA.

In the assay apparatus 110 of the present example, the emission end portion 115BE of the illumination unit 115 is disposed in a state of being in contact with the outer surface of the cover member 10. Then, the light of the illumination unit 115 is guided to the inner edge 18A by being transmitted through the inside of the cover member 10, and the observation region LA is irradiated with the light emitted from the inner edge 18A as the illumination light. Therefore, as shown in Fig. 11, there is less concern that the illumination light is eclipsed by the inner edge 18A. Therefore, the shadow of the cover member 10 is unlikely to be generated in the observation region LA, and the evenness of the light quantity distribution in the observation region LA can be improved.

In addition, in the assay apparatus 110 of the present disclosure, as shown in Fig. 9, the light shielding member 115D as a suppression part for suppressing light leakage that light emitted from the emission end portion 115BE is not incident on the inside of the cover member 10 as the outer shell and leaks to the periphery of the emission end portion 115BE is provided in the emission end portion 115BE of the illumination unit 115. As a result, the utilization efficiency of the light of the illumination unit 115 (the arriving light quantity that arrives in the observation region LA/the emitting light quantity) is increased as compared with a case where the light shielding member 115D is not provided.

In addition, in a case where there is light leakage, it is also conceivable that a part of the light leakage is directly incident on the detection unit 114 without illuminating the observation region LA. Such light directly directed to the detection unit 114 is unnecessary light that causes noise for the detection unit 114. By providing the light shielding member 115D, such unnecessary light can be reduced.

In addition, the light shielding member 115D in the assay apparatus 110 of the present disclosure is a light shielding elastic member that is provided on the entire circumference of the light emission end portion 115BE and that elastically deforms in a case of being pressed against the plane portion 10A which is the outer surface of the cover member 10 that is the outer shell of the case 9. In this way, the deformation of the elastic member makes it possible to fill the gap between the emission end portion 115BE and the plane portion 10A of the cover member 10. Thereby, the effect of suppressing the leakage of the light emitted from the emission end portion 115BE to the outside of the cover member 10 can be enhanced.

In addition, the illumination unit 115 in the assay apparatus 110 of the present disclosure is disposed on both sides (both sides in the X direction of Fig. 8) of the observation window 18 in the plane portion 10A of the outer shell of the cover member 10. Accordingly, as compared with a case where the irradiation is performed from only one side, the effect of suppressing the unevenness of the light quantity distribution in the observation region LA can be enhanced.

The illumination unit 115 does not need to be disposed on both sides of the observation window 18, and may be disposed only on one side. Even in a case where the illumination unit 115 is disposed on one side of the observation window 18, the shadow of the cover member 10 is unlikely to be generated in the observation region LA. As a result, as compared with the prior art shown in Fig. 11, eclipse of light generated in the observation window 18 is suppressed and thus an effect of improving the unevenness of the light quantity distribution in the observation region LA can be expected.

In addition, in the illumination unit 115 in the assay apparatus 110 of the present disclosure, a light emitting diode is used as the light source 115A. As the light source 115A, it is preferable to use a semiconductor light source such as a semiconductor laser in addition to the light emitting diode. The semiconductor light source can be miniaturized as compared with, for example, a light source in which a xenon lamp and a fiber light guide are combined. Therefore, the assay apparatus 110 can be miniaturized. The light source 115A may be an organic electroluminescence (EL) element. Even an organic EL element can be miniaturized as compared with, for example, a light source in which a xenon lamp and a fiber light guide are combined, and thus the assay apparatus 110 can be miniaturized.

In addition, in the cartridge 100 of the present disclosure, a translucent material having a diffusibility to diffuse light is used as the translucent material forming the cover member 10. As the translucent material diffuses the light, the observation region LA is irradiated with the diffused light. Therefore, the effect of suppressing the unevenness of the light quantity distribution incident on the observation region LA can be enhanced as compared with light having a strong straightness.

In addition, in the cartridge 100 of the present disclosure, as shown in Fig. 12A, the diffused light ray transmittance of the translucent material forming the cover member 10 is set to 1% or more and 40% or less in visible light having a wavelength of 420 nm to 680 nm in a case where the transmission distance is 2 mm.

Accordingly, as the illumination light, in addition to the white light, light of various colors such as red light having a center wavelength of about 650 nm and green light having a center wavelength of about 550 nm can be used. As a result, the choice of the color of the illumination light can be increased, and thus, for example, it is easy to select the illumination light of an appropriate color according to the color developed in the assay region L1 or the like included in the observation region LA.

In a case where the diffused light ray transmittance of the translucent material is less than 1%, the light quantity of the illumination light is too small, and a light quantity necessary for detecting the color development state of the observation region LA. On the other hand, in a case where the diffused light ray transmittance of the translucent material is larger than 40%, as indicated by the arrow K8 shown in Fig. 10, the light that is not subjected to a total reflection by the inner surface of the cover member 10 and emitted to the outside of the cover member 10. Such light is light leakage that does not contribute as illumination light in the observation region LA. The light leakage is unnecessary light that is directly incident on the detection unit 114 without passing through the observation region LA. In such a case where the diffused light ray transmittance is more than 40%, unnecessary light due to light leakage increases. In the present example, since the diffused light ray transmittance of the translucent material is 40% or less, unnecessary light due to light leakage can be reduced.

In addition, in the cartridge 100 of the present disclosure, the parallel light ray transmittance of the translucent material forming the cover member 10 is set to 0.1% or less. The lower the parallel light ray transmittance is with respect to the diffused light ray transmittance, the higher the diffusibility of the translucent material is. In addition, the larger the parallel light ray transmittance is, the greater the light emitted straight through the inside of the translucent material and to the outside of the cover member 10 is. As in the present example, the parallel light ray transmittance of the translucent material is preferably 0.1% or less. In a case where the diffused light ray transmittance of the translucent material is 40% or less and the parallel light ray transmittance of the translucent material is 0.1% or less, the straight traveling component can be substantially eliminated while ensuring the diffusibility of the translucent material, and thus unnecessary light due to light leakage can be further reduced.

In addition, the light transmission characteristic of the translucent material of the cover member 10 may be the characteristic shown in Fig. 12B. As shown in Fig. 12B, a diffused light ray transmittance of the translucent material is preferably 0.1% or more and 50% or less in light having at least a specific wavelength in a case where a transmission distance is 2 mm. This specific wavelength is not particularly limited, but in Fig. 12B, as an example, in the red light having a center wavelength of about 650 nm, the light transmission characteristic having a diffused light ray transmittance of 0.1% or more and 50% or less is exhibited. The translucent material having such a light transmission characteristic is, for example, a resin material containing a large amount of a red component.

In this way, in a case where a translucent material having a large diffused light ray transmittance of light having a red wavelength is used, the light quantity necessary as a light quantity for detecting the color development state of the observation region LA can be obtained by using red light as the illumination light. Of course, since the white light includes a red wavelength component, it is also possible to combine such a translucent material with the illumination light of the white light. Since the observation region LA is irradiated with red light, for example, in a case where the assay region develops a color other than red, the color development of the assay region is easy to be confirm. Also in the translucent material having the light transmission characteristic shown in Fig. 12B, the lower limit value of the diffused light ray transmittance is preferably 0.1% or more since the light quantity necessary for detecting the color development state of the observation region LA can be secured, and in a case where the upper limit of the diffused light ray transmittance is less than 50%, unnecessary light due to light leakage can be suppressed.

In addition, in a case where the diffused light ray transmittance of the translucent material is 0.1% or more and 50% or less in the light of the specific wavelength, it is preferable that the parallel light ray transmittance in the light of the specific wavelength is lower than the diffused light ray transmittance, and is 0.5% or less. On the premise that the diffused light ray transmittance of light of a specific wavelength is in the above range, in a case where the parallel light ray transmittance is set to be lower than the diffused light ray transmittance and 0.5% or less, the straight traveling component can be substantially eliminated while ensuring the diffusibility of the translucent material, and thus unnecessary light due to light leakage can be further reduced.

As described above, the specific wavelength shown in Fig. 12B is an example, and may be other than red light, such as green light having a center wavelength of about 550 nm. The specific wavelength is selected according to the color developed in the assay region L1 or the like included in the observation region LA.

### <Modified example 1>

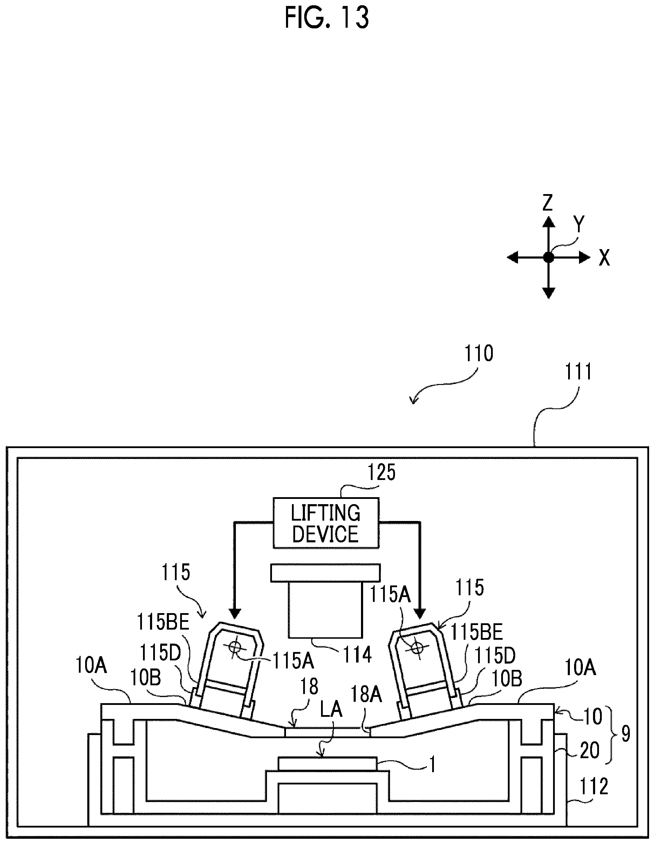

In addition, in the above example, the contact position of the emission end portion 115BE of the illumination unit 115 is set to the plane portion 10A, but as shown in Fig. 13, may be an inclined portion 10B.

By setting the contact position of the emission end portion 115BE to the inclined portion 10B, the observation window 18 and the contact position of the emission end portion 115BE are brought close to each other. The smaller this distance is, the more the loss of light transmitted through the inside of the cover member 10 can be suppressed. Accordingly, it is easy to increase the light quantity that illuminates the observation region LA as compared with a case where the distance is large.

Although the inclined portion 10B is set to a flat surface, the inclined portion 10B may be a curved surface. Even in a case where the inclined portion 10B is a curved surface, since the light shielding member 115D that is capable of being elastically deformed is provided in the emission end portion 115BE, the light shielding member 115D is deformed along the curved surface of the inclined portion 10B, and light leakage from the emission end portion 115BE can be suppressed.

As described above, both the plane portion 10A and the inclined portion 10B are outer surfaces facing the detection unit 114. In this way, the contact position of the emission end portion 115BE is preferably set to an "outer surface facing the detection unit 114" including the plane portion 10A and the inclined portion 10B. By setting the contact position of the emission end portion 115BE to the outer surface facing the detection unit 114, the observation window 18 and the contact position of the emission end portion 115BE are easy to bring close to each other as compared with a case where the emission end portion 115BE is brought into contact with the outer surface "not facing" the detection unit 114. The outer surface not facing is, for example, a side surface of the case 9.

In addition, the example of bringing the contact position of the emission end portion 115BE into contact with the outer surface facing the detection unit 114 is described, but the embodiment of the present disclosure is not limited to this. That is, in a case of being a position where light can be guided to the inner edge 18A of the observation window 18 by transmitting the inside of the outer shell of the case 9, the contact position of the emission end portion 115BE may be an outer surface not facing the detection unit 114, for example, the side surface of the case 9 or the like. In this case, the case body 20 is preferably formed of a translucent material.

### <Modified example 2>

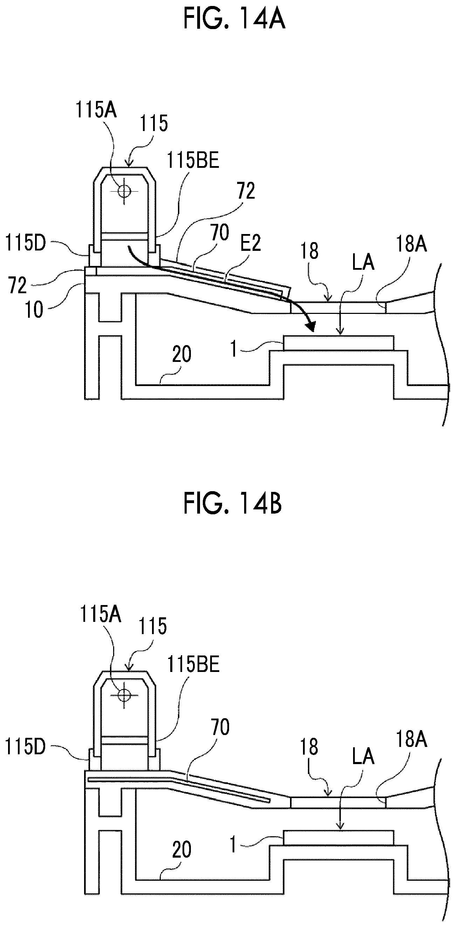

The case 9 of the cartridge 100 according to the present disclosure may include the light guide member 70 shown in Fig. 14A. The light guide member 70 is a member formed of a translucent material having a higher light transmittance than that of the translucent material forming the cover member 10 which is the outer shell of the case 9. The light guide member 70 guides the light incident from the emission end portion 115BE of the illumination unit 115 to the inner edge of the observation window 18 as indicated by an arrow E2.

The transmitted light transmitted through the light guide member 70 is emitted from the inner edge of the observation window 18 and illuminates the observation region LA. Since the light guide member 70 has a higher light transmittance than that of the translucent material forming the outer shell of the cover member 10, the light attenuation is small. Therefore, the light quantity that illuminates the observation region LA can be increased as compared with a case where the light guide member 70 is not provided.

In addition, the light guide member 70 is disposed on an outer surface of an outer shell of the cover member 10. Specifically, the light guide member 70 is disposed from the plane portion 10A to the inclined portion 10B of the cover member 10. Further, in the light guide member 70, the emission end portion 115BE of the illumination unit 115 (more specifically, the light shielding member 115D provided in the emission end portion 115BE) is disposed in contact with a portion disposed on the plane portion 10A of the cover member 10.

In addition, in the light guide member 70, a region other than the contact portion with which the emission end portion 115BE is in contact is shielded from light by the light shielding member 72. The light shielding member 72 is, for example, a light shielding coating material applied to the surface of the light guide member 70. In addition, as the light shielding member 72, a tape or the like having a light shielding property may be used. These light shielding members 72 have at least a lower light transmittance than that of the translucent material forming the outer shell of the cover member 10.

In this way, the light guide member 70 is disposed on the outer surface of the outer shell of the cover member 10, and disposed in contact with illumination unit 115. Therefore, light is directly incident on the light guide member 70 from the illumination unit 115. Accordingly, light can be transmitted to the inside of the light guide member 70 without being affected by the attenuation of light by the translucent material forming the outer shell. Therefore, the light quantity that illuminates the observation region LA can be increased.

In addition, in the light guide member 70, a portion other than the contact portion with which the light shielding member 115D of the illumination unit 115 is in contact is shielded from light by the light shielding member 72. Accordingly, the light incident on the light guide member 70 from the emission end portion 115BE of the illumination unit 115 is prevented from being emitted from a portion other than the contact portion, and thus the utilization efficiency of the light can be improved.

As shown in Fig. 14B, the light guide member 70 may be embedded in a translucent material that forms an outer shell of the cover member 10. In a case where the light guide member 70 is disposed in this manner, the light emitted from the emission end portion 115BE of the illumination unit 115 passes through the translucent material forming the outer shell of the cover member 10 and is incident on the light guide member 70.

In this case, the light is attenuated before the light is incident on the light guide member 70, but after the light is incident on the light guide member 70, the light can be transmitted to the inside of the light guide member 70 without being affected by the attenuation of the light by the translucent material forming the outer shell. Thereby, the utilization efficiency of light can be improved as compared with a case where the light guide member 70 is not provided.

In addition, in the assay apparatus 110 of the present disclosure, as a suppression part for suppressing light leakage that light emitted from the emission end portion 115BE of the illumination unit 115 is not incident on the inside of the outer shell of the cover member 10 and leaks to the periphery of the emission end portion 115BE, the light shielding member 115D is used, but the embodiment of the present disclosure is not limited to this.

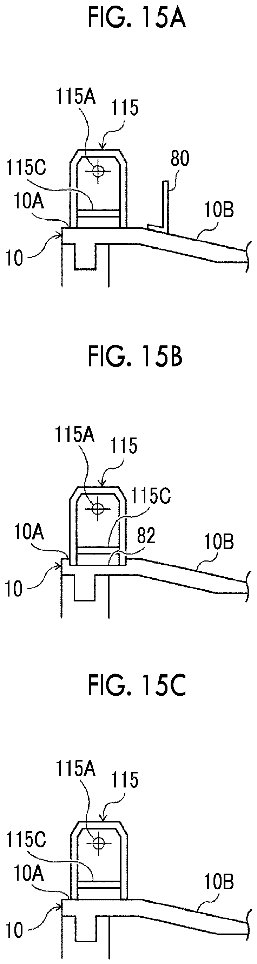

As such a suppression part, for example, the screen 80 shown in Fig. 15A may be used. Light leakage that leaks to the periphery of the emission end portion 115BE may be suppressed by providing the screen 80 in the vicinity of the emission end portion 115BE of the illumination unit 115. In addition, for example, as in the recess 82 shown in Fig. 15B, a portion where the emission end portion 115BE is fitted may be formed on the surface of the cover member 10 to make this recess 82 to function as a suppression part for suppressing light leakage.

In addition, as shown in Fig. 15C, it is not necessary to provide such a suppression part. That is, in a case where the plane portion 10A of the cover member 10 is a flat surface and the surface of the emission end portion 115BE facing the plane portion 10A is also a flat surface having no roughness, light leakage can be suppressed.

In addition, in the above example, the example in which the lifting device 125 is provided as the moving mechanism for moving the illumination unit 115 between the contact position and the retreat position is shown, but the lifting device 125 may not be provided. For example, in the assay apparatus 110, while securing an entry path through which the cartridge 100 enters in a case where the cartridge 100 is loaded, the illumination unit 115 may be spring-biased such that a part of the illumination unit 115 is located within the entry path. In this case, the cartridge 100 traveling along the entry path abuts on the illumination unit 115 in a case where the cartridge 100 is loaded, and while maintaining the abutted state of the illumination unit 115, the illumination unit 115 is moved from the entry path in the direction of retreating against spring-biasing. Since the illumination unit 115 is spring-biased toward the entry path, the contact state between the illumination unit 115 and the cartridge 100 continues while the cartridge 100 is loaded.

In the above-described embodiment, as the processor 120 and a hardware structure of a processing unit as internal configurations thereof that executes various types of processing, such as a detection unit control unit 122, a color development state discrimination unit 123, and a display control unit 124, various processors shown below can be used. The various processors include, for example, a CPU which is a general-purpose processor executing software to function as various processing units as described above, a programmable logic device (PLD), such as a field programmable gate array (FPGA), which is a processor whose circuit configuration can be changed after manufacture, and a dedicated electric circuit, such as an application specific integrated circuit (ASIC), which is a processor having a dedicated circuit configuration designed to perform a specific process.

One processing unit may be configured by one of these various processors, or may be configured by a combination of two or more processors having the same type or different types (for example, a combination of a plurality of FPGAs and/or a combination of a CPU and an FPGA). In addition, a plurality of processing units may be configured with one processor.

As an example in which a plurality of processing units are configured into a single processor, there is a form in which a single processor is configured by a combination of one or more CPUs and software, and this processor functions as a plurality of processing units. A second example of the configuration is an aspect in which a processor that implements the functions of the entire system including a plurality of processing units using one integrated circuit (IC) chip is used. A representative example of this aspect is a system on chip (SoC). In this way, various processing units are configured by one or more of the above-described various processors as hardware structures.

Furthermore, specifically, an electric circuit (circuitry) obtained by combining circuit elements, such as semiconductor elements, can be used as the hardware structure of the various processors. In addition, the present disclosure is not limited to the above embodiments, and can be implemented with appropriate modifications, such as omitting a configuration or replacing a configuration with a different configuration within the scope of the object of the present disclosure.

## Claims

1. An assay apparatus used for immunochromatographic assay, the assay apparatus comprising:
a loading part in which a cartridge is loaded, where the cartridge includes a carrier having an assay region in which a color development state changes depending on whether a sample is positive or negative, and a case in which the carrier is accommodated in an inner space defined by an outer shell formed of a translucent material and in which an opening portion for observing an observation region including the assay region is formed in a part of the outer shell;
an illumination unit that has an emission end portion for emitting light that illuminates the observation region, where the illumination unit makes it possible to dispose at least a part of the emission end portion in a state of being brought into contact, in a position separated from the opening portion on an outer surface of the outer shell of the cartridge in a loaded state, the position being capable of guiding a light incident into the outer shell from the emission end portion to an inner edge of the opening portion by transmitting the incident light through the outer shell; and
a detection unit that is disposed at a position facing the opening portion of the cartridge in a loaded state and detects a color development state of the assay region, where the detection unit optically detects the color development state by receiving a reflected light which is emitted from an inner edge of the opening portion and reflected by a surface of the observation region.

2. The assay apparatus according to claim 1,
wherein in the emission end portion, a suppression part for suppressing light leakage, in which light emitted from the emission end portion is not incident on the outer shell and leaks to the periphery of the emission end portion, is provided.

3. The assay apparatus according to claim 2,
wherein the emission end portion has an emission window that emits light and a window frame that surrounds the periphery of the emission window, and
the suppression part is a light shielding elastic member that is provided on the window frame and elastically deforms in a case of being pressed against the outer surface of the outer shell.

4. The assay apparatus according to any one of claims 1 to 3,
wherein a contact position of the emission end portion is an outer surface facing the detection unit in the outer shell.

5. The assay apparatus according to claim 4,
wherein the assay apparatus has at least two illumination units, and the two illumination units are disposed on both sides of the opening portion in the outer shell one by one.

6. The assay apparatus according to any one of claims 1 to 5,
wherein a light source of the illumination unit is a semiconductor light source.

7. A cartridge to be loaded in the assay apparatus according to any one of claims 1 to 6,
wherein, as the translucent material, a translucent material having a diffusibility to diffuse light is used.

8. The cartridge according to claim 7,
wherein a diffused light ray transmittance of the translucent material is 0.1% or more and 50% or less in light having at least a specific wavelength in a case where a transmission distance is 2 mm.

9. The cartridge according to claim 7,
wherein a diffused light ray transmittance of the translucent material is 1% or more and 40% or less in light having a wavelength of 420 nm to 680 nm in a case where a transmission distance is 2 mm.

10. The cartridge according to any one of claims 7 to 9,
wherein the case includes a light guide member that is formed of a translucent material having a higher light transmittance than the translucent material forming the outer shell, where the light guide member guides light incident from the emission end portion to the inner edge of the opening portion.

11. The cartridge according to claim 10,
wherein the light guide member is disposed on the outer surface of the outer shell and is disposed in contact with at least a part of the emission end portion of the illumination unit, and
in the light guide member, a region other than a contact portion to be brought in contact with the emission end portion is shielded from light.
